Europäisches Patentamt

European Patent Office

Office européen des brevets

(19)

(11) Veröffentlichungsnummer: **0 273 032**
**B1**

(12) **EUROPÄISCHE PATENTSCHRIFT**

(45) Veröffentlichungstag der Patentschrift: **13.06.90**

(51) Int. Cl.⁵: **G 01 B 11/02**

(21) Anmeldenummer: **86904778.7**

(22) Anmeldetag: **08.08.86**

(86) Internationale Anmeldenummer:
**PCT/DE86/00322**

(87) Internationale Veröffentlichungsnummer:
**WO 87/01195 26.02.87 Gazette 87/05**

(54) **VERFAHREN ZUR ERMITTLUNG EINER GEOMETRISCHEN KENNGRÖSSE FÜR GEKRÄUSELTE, IRREGULÄR STRUKTURIERTE FASERN.**

(30) Priorität: **14.08.85 DE 3529137**
**30.07.86 DE 3625791**

(43) Veröffentlichungstag der Anmeldung:
**06.07.88 Patentblatt 88/27**

(45) Bekanntmachung des Hinweises auf die
Patenterteilung:
**13.06.90 Patentblatt 90/24**

(84) Benannte Vertragsstaaten:
**AT BE CH DE FR GB IT LI LU NL SE**

(56) Entgegenhaltungen:
**EP-A-01 608 95**
**GB-A-21 484 98**

**Messen + Prüfen / Automatik, Oktober 1983,
"Automatisches Bildanalysesystem", pages 546-548, 556**

(73) Patentinhaber: **FRAUNHOFER-GESELLSCHAFT
ZUR FÖRDERUNG DER ANGEWANDTEN
FORSCHUNG E.V.
Leonrodstrasse 54
D-8000 München 19 (DE)**
(84) **AT BE CH DE FR GB IT LI LU NL SE**

(73) Patentinhaber: **B.A.T. Cigaretten-Fabriken
GmbH
Alsterufer 4
D-2000 Hamburg 36 (DE)**
(84) **GB**

(72) Erfinder: **MELCHIOR, Klaus
Löchgauerstr. 8
D-7122 Besigheim (DE)**
Erfinder: **RUEFF, Manfred
August Lämmle Allee 51
D-7064 Geradstetten (DE)**
Erfinder: **FEDERLE, Hartmut
Rantzaustr. 48
D-2070 Ahrensburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein Verfahren zur Ermittlung einer geometrischen Kenngröße für eine Gemisch gekräuselter, irregulär strukturierter Fasern unterschiedlicher Länge und Breite wie Textilfasern und Tabakfasern mit den Merkmalen der im Oberbegriff des Patentanspruchs 1 bezeichneten Gattung.

Eine geometrische Kenngröße für gerade Fasern, die eine einheitliche Dicke, jedoch eine unterschiedliche Länge aufweisen, ist die mittlere Faserlänge. Diese kann so ermittelt werden, daß eine Menge von Fasern auf einer abgegrenzten Fläche einer Unterlage vereinzelt wird, mittels einer Kamera ein Bild der vereinzelten Fasern erzeugt wird, aus dem Bild die Länge der Einzelfasern vermessen wird, die Längen der Einzelfaser addiert und die Summe durch die Anzahl der Fasern geteilt wird. Die so erhaltene geometrische Kenngröße ist die mittlere Faserlänge (s. z.B. "Messen + Prüfen, Automatik", Oktober 1983, S. 546 bis 548 und S. 556). Alle diese Verfahren werden mit einem Bild pro Messung durchgeführt.

Bei gekräuselten Fasern mit einheitlichem Faserdurchmesser, wie z.B. bei Textilfasern und bei gekräuselten Fasern mit unterschiedlicher Faserbreite und Verzweigungen der Einzelfaser, wie z.B. bei Tabakfasern, ist die mittlere Faserlänge als geometrische Kenngröße zur Kennzeichnung eines Fasergemisches nicht geeignet, weil eine mittlere Faserlänge nicht bestimmbar ist und weil Verzweigungen, unterschiedliche Faserbreiten längs einer Einzelfaser und die Faserkräuselung unberücksichtigt bleiben.

Der Erfindung liegt deshalb die Aufgabe zugrunde, ein Verfahren gemäß dem Oberbegriff des Patentanspruchs 1 zu schaffen, durch das eine geometrische Kenngröße für gekräuselte, irregulär strukturierte Fasern in einem Fasergemisch mit hoher Faseranzahl ermittelbar ist, die Fasern mit irregulärer Faserstruktur in Kräuselung Länge, Breite und Durchmesser kennzeichnen kann.

Diese Aufgabe wird erfindungsgemäß durch die im Anspruch 1 aufgeführten Verfahrensschritte gelöst. Eine weitere Ausgestaltung der Erfindung ist im Anspruch 2 beschrieben.

Die Vorteile der Erfindung bestehen insbesondere darin, daß durch das Herstellen zweier Bilder und die anschließende Rasterbildung und die Verschiebung dieser Raster gegeneinander die Bildung einer Kenngröße zur Kennzeichnung eines Fasergemisches ermöglicht wird, die die Beurteilung und Klassifizierung dieses Fasergemisches nach dessen Eigenschaften zuläßt, die z.B. für die Qualitätskontrolle des Fasergemisches von entscheidender Bedeutung sind.

Kurze Beschreibung der Zeichnungen Die Erfindung wird anhand der Zeichnungen näher erläutert. Es zeigen

Fig. 1 Bild einer vereinzelten Fasermenge;

Fig. 2 Bild einer vereinzelten Fasermenge, mit einem darübergelegten transparenten identischen Bild, das um einen Rasterschritt (ca. 1 mm) in (+x)-Richtung verschoben ist;

Fig. 2a Vergrößerte und schematische Darstellung der Verschiebung in (x)- bzw. (+y)- bzw. (x/±y)-Richtung um jeweils einem Rasterschritt $x_o$, $y_o$;

Beschreibung des Koordinatensystems, Angaben der Rasterschritte $\Delta x_o$, $\Delta y_o$;

Fig. 2b Musterbild in Ausgangsposition; gestrichelte Fläche: Zu erfassende Fläche F;

Fig. 2c Musterbild und verschobenes Bild. Verschiebung um $\Delta x_o$ in +x-Richtung gestrichelte Fläche: Bei Verschiebung um eine Einheit ($\Delta x_o$) in +x-Richtung zu erfassende Fläche;

Fig. 2d Musterbild und verschobenes Bild. Verschiebung um $\Delta y_o$ in +y-Richtung; gestrichelte Fläche: Bei Verschiebung um eine Einheit ($\Delta y_o$) in +y-Richtung zu erfassende Fläche;

Fig. 2e Musterbild und verschobenes Bild; Verschiebung um $\Delta y_o$ in +y-Richtung und um $\Delta x_o$ in +x-Richtung; gestrichelte Fläche: Zu erfassende Fläche;

Fig. 2f Musterbild und verschobenes Bild. Verschiebung um $\Delta y_o$ in −y-Richtung und um $\Delta x_o$ in +x-Richtung; gestrichelte Fläche: Zu erfassende Fläche.

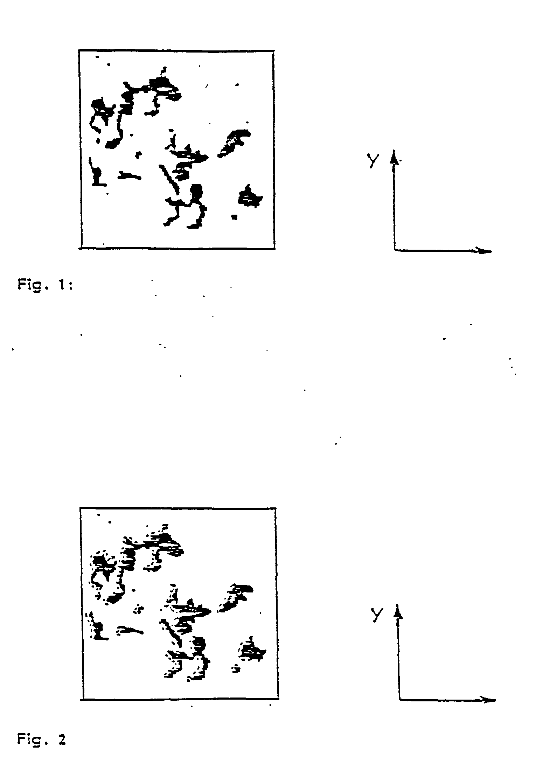

In Figur 1 ist ein Bild, z.B. ein Diabild, einer Menge von Fasern (Tabakfasern) dargestellt, die auf einer abgegrenzten Fläche von 5 cm × 5 cm vereinzelt angeordnet sind, so daß keine Überschneidungen von Einzelfasern vorkommen. Die Einzelfasern sind gekräuselt und haben unterschiedliche Faserlängen, unterschiedliche Faserdicken längs einer Einzelfaser, die bei Tabakfasern von Blattrippen kommen können und Verzweigungen. Mittels eines daruntergelegten feinen Rasters werden die Rasterelemente mittels einer Zählvorrichtung ausgezählt, die von den einzelnen Fasern überdeckt werden, und addiert. Die Summe ergibt die von den Einzelfasern überdeckte Fläche F, gemessen in Rastereinheiten.

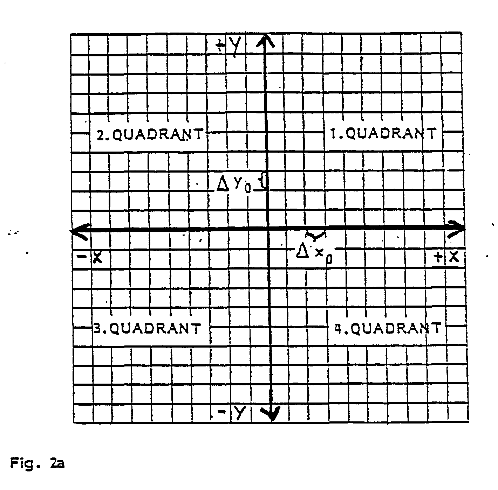

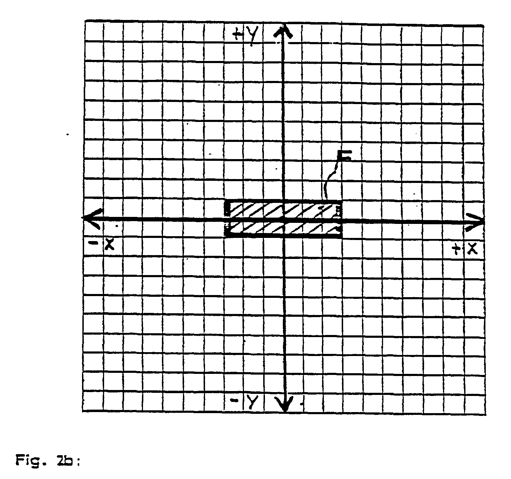

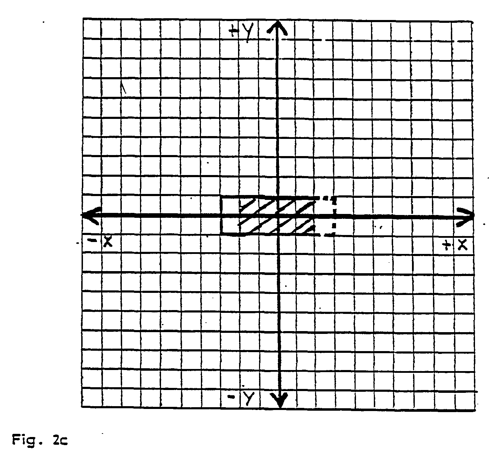

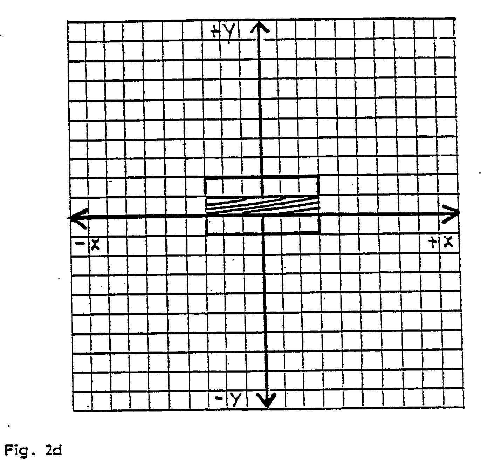

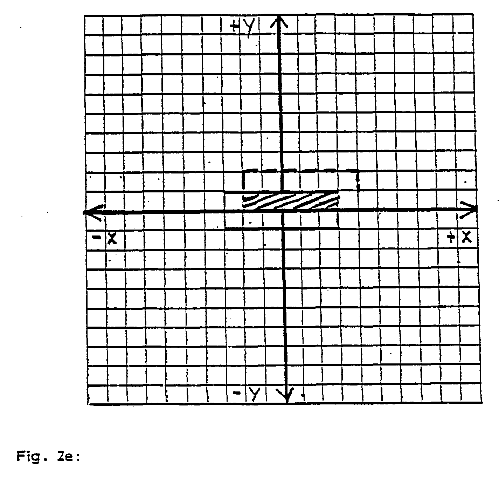

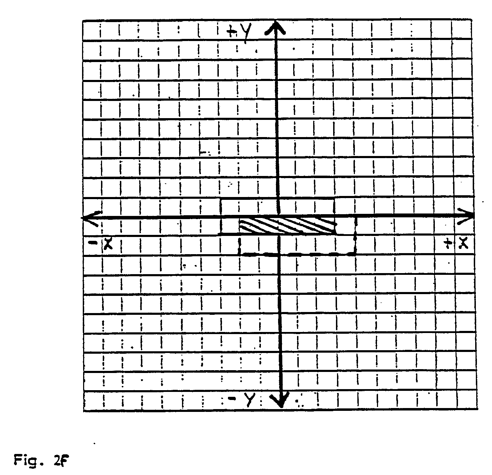

In Fig. 2 ist das Diabild gemäß Fig. 1 mit durchgezogenen Strichen dargestellt. Auf dieses Diabild ist ein identisch gleiches Diabild gelegt (eine notwendige Voraussetzung für das hier dargestellte Meßverfahren), das um einen Rasterschritt in (+x)-Richtung verschoben ist. Dieses ist gestrichelt gezeichnet. Gemäß Verfahrensschritt c) von Anspruch 1 werden für jede Einzelfaser die Rasterelemente mittels einer Zählvorrichtung ermittelt, die von beiden Dias überdeckt werden. In ähnlicher Weise werden die Rasterelemente ermittelt sowohl für in (+y)-Richtung als auch diagonal in (x/y)-Richtung um je eine Rastereinheit verschobene Diabilder.

Die sich bei allen diesen Verschiebungen überdeckenden Rasterelemente werden addiert und zur Fläche F hinzuaddiert. So entsteht eine neue Fläche F'>F. Zur Normierung wird diese Fläche F' durch F dividiert, die bei unverschobenem Bild ermittelt wurde. Die Flächen F und F' werden in Rastereinheiten gemessen. Hierdurch ergibt sich die geometrische Kenngröße zur Kennzeichnung von gekräuselten, irregulär strukturierten Fasern gemäß der Erfindung. Zur noch genaueren Ermittlung der Kenngröße kann die Verschiebung um mehrere Rasterschritte in (+x)- bzw. (−y)- bzw. (x/

±y)-Richtung erfolgen, wobei jeweils die sich noch überdeckenden Rasterelemente mittels einer Zählvorrichtung ermittelt und zur Fläche F hinzuaddiert werden.

Aus Versuchen hat sich gezeigt, daß mit zwei Verschiebungen in den jeweiligen Richtungen eine ausreichend genaue Kenngröße erhalten werden kann. Dabei ist der Zahlenwert der Kenngröße umso größer, je größer die Anzahl der Rasterschritte ist. Kenngrößen unterschiedlicher Schrittzahl sind deshalb nicht vergleichbar.

Es ist vorteilhaft, ein quadratisches Raster zu wählen, wobei eine Rasterschritt höchstens der kleinsten Faserbreite einer Einzelfaser entspricht.

In Weiterbildung der Erfindung werden gemäß Anspruch 2 vorteilhaft bekannte Bildverarbeitungsgeräte zur Ermittlung der Kenngröße eingesetzt, die sehr feine Rasterschritte ermöglichen. Diese bestehen aus eine elektronischen Kamera, die ein optoelektronisches Rasterbild erzeugt. Die von den Einzelfasern überdeckten Rasterelemente werden in Speicher eines Digitalrechners digital abgelegt. Die Verschiebungen der Rasterbilder um (x), (+y), (x/±y), (x/±2y), (2x), (2x/±y), (2x/±2y), (2y) werden in Digitalrechner vorgenommen, wie die Ermittlung der sich überdeckenden Rasterelemente, und die Berechnung der geometrischen Kenngröße gemaß der Verfahrensschritte b), c), d) von Anspruch 1. Hierzu eignen sich handelsübliche Bildverarbeitungsgeräte.

Die Verschiebung von identischen Bildern gegeneinander und die Ermittlung der sich noch überdeckenden Rasterelemente entspricht einem Autokorrelationsverfahren.

Bei Tabakfasern wurden zur Ermittlung der geometrischen Größe folgende Versuche Durchgeführt:

Es wurden Zigaretten von unterschiedlichen Zigarettenmarken geöffnet. Von einer einzelnen Zigarette wurde eine Teilmenge Tabak auf einer ebenen Fläche mit den Abmessungen 5 cm × 5 cm vereinzelt. Mittels eines Bildverarbeitungssystems wurden die Verfahrensschritte b), c), d) gemäß Anspruch 1 durchgeführt. Dabei wurde erstens zur stärkeren Betonung der Längenausdehnung eine Gewichtung (erstes Moment der Autokorrelationsfunktion) der einzelnen Überdeckungsflächen mit der Größe des Schrittes vorgenommen und zweitens auch die Verschiebungen im zweiten und dritten Quadranten durchgeführt (s, Abb. 2a). So ergaben sich bei zwei Rasterschritten folgende Kenngrößen:

| Zigarettenmarke | Kennzahl |
|:---:|:---:|
| I | 18,3222 |
| II | 17,8687 |
| III | 16,0040 |
| IV | 16,2076 |
| V | 17,2862 |
| VI | 17,8863 |
| VII | 18,1727 |
| VIII | 18,4303 |
| IX | 18,8275 |
| X | 18,9407 |
| XI | 20,0150 |

Die Kenngrößen der einzelnen Zigaretten sind deutlich verschieden. Durch ergänzende Versuche kann festgestellt werden, wie unterschiedliche Kräuselungen, unterschiedliche Überdeckungsflächen von Einzelfasern, unterschiedliche Breiten der Einzelfasern (bei Tabakfasern Rippen und Verzweigungen) die Kennzahl beeinflussen.

Bei geraden Einzelfasern mit gleichem Querschnitt längs der Einzelfaser kann die sich nach dem Verfahren der Erfindung ergebende Kennzahl im wesentlichen der mittleren Faserlänge zugeordnet werden.

**Patentansprüche**

1. Verfahren zur Ermittlung einer geometrischen Kenngröße für ein gemisch von Fasern unterschiedlicher Länge und Breite, wobei eine Menge von Fasern auf einer abgegrenzten Fläche einer Unterlage vereinzelt wird, mittels einer Kamera ein Bild der vereinzelten Fasern erzeugt wird, aus dem Bild durch Ermittlung von Meßwerten geometrische Kenngrößen der Fasern gewonnen werden, dadurch gekennzeichnet,

a) daß zwei deckungsgleiche Bilder erzeugt werden,

b) daß danach mittels eines Rasters, die Rasterelemente (F) ermittelt werden, die von den Fasern überdeckt werden, daß dabei das Raster so gewählt ist, daß ein Rasterschritt höchstens so breit ist wie die dünnste Stelle einer Einzelfaser.

c) daß ferner die Bilder dann mindestens um einen Rasterschritt oder um mehrere Rasterschritte in einer Richtung (x) gegeneinander verschoben werden und die Anzahl der Rasterelemente mit Fasern der beiden Bilder, die sich nach der Verschiebung noch gegenseitig überdecken, mittels einer Zählervorrichtung ermittelt wird, daß anschließend die Bilder von der Ausgangslage in einer dazu senkrechten Richtung (+x) sowie daran anschließend in (x/±y)-Richtung, also diagonal, um die gleiche Strecke bzw. Strecken verschoben werden und die Anzahl der Rasterelemente mit Fasern der beiden Bilder mittels einer Zählervorrichtung ermittelt wird, die sich nach den jeweiligen Verschiebungen noch gegenseitig überdecken,

d) die nach b) und c) ermittelten Rasterelemente addiert und durch die Anzahl der nach b) ermittelten Rasterelemente (F) dividiert werden, wodurch sich die Kenngröße ergibt.

2. Verfahren nach Anspruch 1, dadurch gekennzeichnet, daß als optische Kamera eine elektronische Kamera vorgesehen ist, die ein optoelektronisches Rasterbild erzeugt, daß das Rasterbild digital im Speicher eines Digitalrechners gespeichert wird und daß die Verfahrensschritte b), c), d) gemäß Anspruch 1 vom Digitalrechner durchgeführt werden.

**Revendications**

1. Procédé de déterminer une grandeur caractéristique géométrique d'un mélange de fibres aux longueurs et largeurs différentes, dans lequel une quantité de fibres est dispersée sur une surface limitée d'un support, et dans lequel une image des fibres dispersées est faite au moyen d'un appareil photographique, pendant que des valeurs mesurées sont obtenues pour l'extraction des grandeurs caractéristiques géométriques des fibres de ladite image, caractérisé en ce

(a) que deux images égales sont faites,

(b) que çi-après, au moyen d'un réseau, les éléments du réseau (F) sont déterminés qui sont recouverts par les fibres, à une telle choix du réseau qu'un pas du réseau présente une largeur maximale qui correspond à la plage la plus mince d'une fibre isolée,

(c) qu'en plus les images sont ensuite déplacées les unes par rapport aux autres par au moins un pas du réseau ou par plusieurs pas du réseau en un sens (x), et que le nombre des éléments du réseau aux fibres desdites deux images, qui s'encore recouvrent réciproquement après le déplacement, est déterminé au moyen d'un appareil compteur, que çi-après les images sont déplacées de la position de départ en un sens y orthogonal (+x) et ensuite en sens (x/+y), c'est-à-dire en diagonale, par une distance égale ou par des distances égales, et qu' au moyen d'un appareil compteur le nombre des éléments du réseau aux fibres des deux images est déterminé qui s'encore recouvrent réciproquement après les déplacements respectifs,

(d) que les éléments du réseau déterminés selon les opérations (b) et (c) sont additionnés et divisés par le nombre des éléments du réseau (F) déterminé selon l'opération (b) pour obtenir la grandeur caractéristique.

2. Procédé selon la Revendication 1, caractérisé en ce qu'une caméra électronique est pourvue comme appareil photographique, qui produit une trame opto-électronique, que ladite trame est mémorisée en forme numérique dans la mémoire d'un calculateur numérique, et en ce que les opérations (b), (c), (d) selon la Revendication 1 sont exécutées par ledit calculateur numérique.

**Claims**

1. Process for determining a geometrical parameter for a mixture of fibres of different length and width, a quantity of fibres being separated on a clearly defined surface of a substrate, an image of the separated fibres is produced by means of a camera and geometrical parameters of the fibres are obtained from the image by determining the measured values, characterized in that

a) two congruent images are produced,

b) by means of a grid, the grid elements (F) are determined which are covered by the fibres, the grid being selected in such a way that one grid step is at the most as wide as the thinnest point of a filament,

c) the images are then reciprocally displaced by at least one grid step or several grid steps in one direction (x) and the number of grid elements with fibres of the two images, which still reciprocally coincide after the displacement, is determined by means of a counter, subsequently the images are moved from the starting position in a direction (+x) at right angles thereto and then subsequently in a (x/±y) direction, i.e. diagonally by the same distance or distances and by means of a counter the number of grid elements with fibres of the two images which still reciprocally coincide after the displacements is determined and

d) the grid elements determined according to b) and c) are summated and divided by the number of grid elements (F) determined according to b) and as a result the parameter is obtained.

2. Process according to claim 1, characterized in that the optical camera is constituted by an electronic camera, which produces an optoelectronic grid image, that the grid image is ditigally stored in the memory of the digital computer and that process steps b), c), d) according to claim 1 are performed by the digital computer.

Fig. 1:

Fig. 2

Fig. 2a

Fig. 2b :

Fig. 2c

Fig. 2d

Fig. 2e:

Fig. 2F